# EUROPEAN PATENT SPECIFICATION

(11) **EP 0 311 383 B1**
(45) Date of publication and mention of the grant of the patent: **06.10.1993**
(21) Application number: 88309292.6
(22) Date of filing: 06.10.1988
(51) Int. Cl.: C12P 21/08, G01N 33/94, G01N 33/577

(54) **Monoclonal antibody to methamphetamine, preparation of the same, assay method and assay kit of methamphetamine**
Monoklonaler Antikörper gegen Methamphetamin, seine Herstellung, Testverfahren und Testsatz für Methamphetamin
Anticorps monoclonal de méthamphétamine, sa préparation, méthode d'essai et trousse d'essai de métamphétamine

(30) Priority: 09.10.1987 JP 253781/87; 04.03.1988 JP 49489/88; 04.03.1988 JP 49490/88; 24.06.1988 JP 154608/88
(43) Date of publication of application: 12.04.1989
(73) Proprietor: UBE INDUSTRIES, LTD., Ube-shi, Yamaguchi-ken 755 (JP)
(72) Inventor: Uda, Taizo, Ube-shi Yamaguchi-ken (JP); Itoh, Yukikatsu, Ube-shi Yamaguchi-ken (JP); Usagawa, Takashi, Ube-shi Yamaguchi-ken (JP); Nishimura, Minoru, Ube-shi Yamaguchi-ken (JP); Hifumi, Emi, Ube-shi Yamaguchi-ken (JP); Hongyo, Kenichi, Ube-shi Yamaguchi-ken (JP)
(74) Representative: Brock, Peter William

(56) References cited:
- EP-A- 0 044 441
- EP-A- 0 101 912
- CHEMICAL ABSTRACTS, vol. 98, no. 21, 23rd May 1983, page 209, abstract no. 174225r, Columbus, Ohio, US; AOKI, KIMIKO et al.: "Enzyme immunoassay for methamphetamine", & J. PHARMACOBIO-DYN. 1983, 6(1), 33-8
- CHEMICAL ABSTRACTS, vol. 108, no. 1, 4th January 1988, page 164, abstract no. 1690k, Columbus, Ohio, US; IWASAKI, MINORU: "Forensic immunochemical studies of methamphetamine. Preparation of a specific antibody to methamphetamine", & NIPPON HOIGAKU ZASSHI 1987, 41(3), 217-23
- CHEMICAL ABSTRACTS, vol. 112, no. 3, 15th January 1990, page 405, abstract no. 19857n, Columbus, Ohio, US; TAKEYA, MAKOTO et al.: "Monoclonal antibodies to methamphetamine or its derivatives", & JP-A-01 071 480 (16-03-1989)
- CHEMICAL ABSTRACTS, vol. 112, no. 3, 15th January 1990, page 405, abstract no. 19858p, Columbus, Ohio, US; TAKEYA MAKOTO et al.: "Monoclonal antibodies to methamphetamine or its derivatives", & JP-A-01 071 481 (16-03-1989)
- J. Pharm. Dyn (1983) 6, 33-38

## Description

### BACKGROUND OF THE INVENTION

This invention relates to a monoclonal antibody to methamphetamine which is kind of antihypnotics and a method for preparing the monoclonal antibody. This invention further relates to an assay method and an assay kit of methamphetamine.

Recently, abuse of antihypnotics is becoming a great social problem. In spite of severe regulation by the police, the present time has become the secondary abuse era, and the number of inspections per year has been estimated to reach as high as 40,000 cases.

With increase of antihypnotic case criminals, it has been increasingly demanded to grasp those who are committing abuse of antihypnotics such as methamphetamine (hereinafter abbreviated to as "MA") simply, rapidly and accurately.

As the method for solving such problems, an immunological assay method by use of a monoclonal antibody exhibiting very high specific reactivity with MA, etc. has been considered to be an excellent method, but no report concerning preparation of a monoclonal antibody to MA has been recognized up to date.

As the presently available method for analyzing of drugs or poisons such as MA, there have been developed (1) analysis by chromatography, (2) analysis by spectrometry, (3) analysis by immunoassay, etc. depending on the respective purpose. However, although the amount of MA can be grasped at high sensitivity and accurately according to the analysis of (1) and (2), a relatively large amount of sample is required to be synthesized for enhancing assay sensitivity together with cumbersome extraction operations during prepareation of the sample, and further a large scale analysis by use of a antiserum (polyclonal antibody) to MA of (3), a large number of test samples can be analyzed at one time within a short time but due to use of a polyclonal antibody, sensitivity is poor, and also specificity for MA is not sufficient, involving particularly the problem of having cross-reactivity with ephedrine or methylephedrine contained in medicines for cold [e.g., K. Aoki et al; J. Pharm. Dyn. vol. 6, p. 33 (1983), S Tokura; Anal. Biochem, vol. 161, p. 117 (1987)].

The method by use of gas chromatography or gas chromatography-mass spectrometry has been considered as an excellent method which can quantitate accurately a very minute amount of MA. However, in these methods, since the sample to be used for assay is requied to be subjected bo pre treatement by solvent extraction or adsorption chromatography, the operations before assay are cumbersome, and there is also the problem that there is needed the time for such operation.

The method utilizing the Simon reaction specific for secondary amines, but for assay of only MA, there is the problem that specificity for MA is low.

As the method for solving such problems, the immunological assay method by use of a monoclonal antibody exhibiting very high specific reactivity with MA has been considered to be an excellent method but there has heretofore been recognized no report concerning the assay method and the assay kit by use of a monoclonal antibody having very high specific immunoreactivity with methamphetamine.

### SUMMARY OF THE INVENTION

An object of the present invention is to provide a monoclonal antibody exhibiting very high specific reactivity with MA which is an antihypnotic, a method for preparing the monoclonal antibody, a method for assay MA and a assay kit by use thereof.

Another object of the present invention is to provide a kit for assay MA comprising essentially a carrier having a monoclonal antibody having very high specific immunoreactivity with MA which is an antihypnotic carried thereon and a carrier having MA carried thereon, and a method for assay MA by use of the assay kit.

The present inventors, as the result of intensive study in order to solve the above problems, have found that a monoclonal antibody exhibiting very high specific reactivity with methamphetamine (MA) which is an antihypnotic can be obtained by culturing a cell strain obtained by immunization of an animal, to accomplish the present invention.

Thus, the present invention concerns a monoclonal antibody to methamphetamine as deposited at the Fermentation Research Institute and assigned accession number FERM BP-1494, having the following cross reactivities expressed in terms of reactive ratios relative to methamphetamine:-
methamphetamine 1, amphetamine 0.0051, ephedrine 0.0018, methylephedrine 0.022, methoxyphenamine 0.023, phentermine 0.042, norephedrine <0.001, N,N'-dibenzylethylenediamine 0.17, p-hydroxymethamphetamine 0.0039, p-hydroxyamphetamine <0.001, p-hydroxyephedrine <0.001, p-hydroxynorephedrine <0.001, mescaline <0.00001;
a monoclonal antibody to methamphetamine as deposited at the Fermentation Research Institute and assigned accession number FERM BP-1495, having the following cross reactivities expressed in terms of reactive ratios relative to methamphetamine:-
methamphetamine 1, amphetamine 0.0062, ephedrine 0.0025, methylephedrine 0.028, methoxyphenamine 0.0037, phentermine 0.12, norephedrine <0.001, N,N'-dibenzylethylenediamine 0.23, p-hydroxymethamphetamine 0.14, p-hydroxyamphetamine 0.003, p-hydroxephedrine <0.001, p-hydroxynorephedrine <0.001, mescaline <0.00001; and
a monoclonal antibody to methamphetamine as deposited at the Fermentation Research Institute and assigned accession number FERM BP-1496, having the following cross reactivities expressed in terms of reactive ratios relative to methamphetamine:-
methamphetamine 1, amphetamine 0.019, ephedrine 0.0012, methylephedrine 0.016, methoxyphenamine 0.0016, phentermine 0.0039, norephedrine <0.001, N,N'-dibenylethylenediamine 0.0055, p-hydroxymethamphetamine 0.014, p-hydroxyamphetamine <0.001, p-hydroxyephedrine 0.0031, p-hydroxynorephedrine <0.001, mescaline <0.0001.

The monoclonal antibodies to MA are produced by a cell strain obtainable by immunization of an animal
Further, the present invention concerns a method for producing a monoclonal antibody to MA, which comprises culturing a cell strain producing a monoclonal antibody to MA.

Further, the present invention concerns a method for assay methamphetamine which comprises allowing a methamphetamine-bound macromolecular protein immobilized on a solid phase support and methamphetamine in a sample to be assayed to react competitively with a reagent comprising a monoclonal antibody having very high specific immunoreactivity with methamphetamine labeled with an enzyme, and a methamphetamine assay kit to be used in the method for assay methamphetamine, comprising essentially:
(a) a methamphetamine-bound macromolecular protein; and
(b) a reagent comprising monoclonal antibody having a very high specific immunoreactivity with methamphetamine labeled with an enzyme.

The present invention also concerns a method for assay methamphetamine, which comprises immobilizing a monoclonal antibody having a very high specific immunoreactivity with methamphetamine on a solid phase support, and allowing an enzyme-labeled methamphetamine and methamphetamine in a sample to be assayed to react competitively with the monoclonal antibody immobilized on the solid phase support, and a methamphetamine assay kit to be used in the method for assay methamphetamine, comprising essentially:
(a) a monoclonal antibody having a very high specific immunoreactivity with methamphetamine; and
(b) an enzyme-labeled methamphetamine.

The present invention further concerns a methamphetamine assay kit to be used for assay methamphetamine, comprising essentially:
(a) a carrier having a monoclonal antibody having a very high specific immunoreactivity with methamphetamine carried thereon; and
(b) a carrier having methamphetamine carried thereon, and a method for assay methamphetamine, which comprises allowing methamphetamine in a sample to be assayed and the methamphetamine carried on the carrier to react competitively with the monoclonal antibody carried on the carrier by use of the methamphetamine assay kit as defined above.

### BRIEF DESCRIPTION OF THE DRAWINGS

Fig. 1 shows the standard curve of MA prepared by use of "enzyme-labeled antibody".

Fig. 2 shows the results of the respective reactivities of MA, amphetamine (AP), methylephedrine (ME), methoxyphenamine (MPA) prepared by use of "enzyme-labeled antibody".

Fig. 3 shows the standard curve of MA prepared by use of enzyme-labeled MA.

### DESCRIPTION OF THE PREFERRED EMBODIMENTS

The present invention will be described in detail below.

The monoclonal antibody of the present invention is produced by a cell strain obtained by an immunized animal, and has very high specific reactivity with MA which is an antihypnotic.

The immunogen to be used for immunization of an animal in the present invention is not particularly limited, provided that it can obtain a monoclonal antibody having very high specific reactivity with MA. For example, there may be included MA, salts of MA and those having MA bound to a carrier with a molecular weight of 10,000 or higher, but it is preferred to use one having MA bound to a carrier with a molecular weight of 10,000 or higher. As the carrier having a molecular weight of 10,000 or higher to be used at this time, there may be included biological macromolecules such as bovine serum albumin, ovalbumin, key-hole limpet hemocyanin, immunoglobulin, etc.

The monoclonal antibody of the present invention can be also obtained by culturing a hybridoma strain such as the MA-7 strain (FERM-P 1494), MA-13 strain (FERM-P 1495) or MA-15 strain (FERM-P 1496) which are hybridomas obtained by fusion of the lymphocytes obtained from an immunized mouse and myeloma cells of mouse by the present inventors.

Preparation of such hybridomas can be performed according to the method known in the art, for example, the method of Milstein and Koehler [Nature, vol. 256, p. 495 (1976)]. The outline of the preferred method for preparing such hybridoma strain is to be described successively below.

### Preparation of hybridoma strain producing monoclonal antibody

### (i) Preparation of immunogen and antibody for analysis:

Immunogen can be prepared by converting a compound such as MA, salts of MA, etc. into an N-(4-aminobutyl) derivative by use of N-(4-bromobutyl)phthalimide, and then binding it to a carrier having a molecular weight of 10,000 or higher [for example, preferably biological macromolecules such as bovine serum albumin (BSA), ovalbumin (OVA), key-hole limpet hemocyanin (KLH)] with the use of a carbodiimide such as 1-cyclohexyl-3-(2-morpholinoethyl)carbodiimide metho-p-toluenesulfonate (hereinafter abbreviated to as "CMEC"), 1-ethyl-3-(3-dimethylaminopropyl)carbodiimide hydrochloride (hereinafter abbreviated to as "EDPC"), etc. On the other hand, as the antigen for analysis, one prepared similarly as in the preparation method of immunogen by use of a carrier different from that in preparation of the immunogen is used.

### (ii) Preparation of immunized animal lymphocytes:

The method for immunizing an animal (e.g. mouse, rat, etc.) can be performed by administering the immunogen of the above (i) (10 to 400 µg) dissolved in PBS (Dulbecco's phosphate buffered saline) to an animal once or several times with intervals of several weeks.

Immunication for the first time can be also conducted without administering an adjuvant (immunization promoting substance containing alum, dead tuberclosis bacterial cells, nucleic acid, etc.), but it is preferred to administer an emulsion prepared by use of an adjuvant.

Lymphocytes can be obtained from blood, lymph node, spleen, etc. several days after the final immunization after confirmation of sufficient antibody value of the immunized animal, but preferably from spleen.

### (iii) Preparation of myeloma cells:

For cell fusion, myeloma cells such as MPC-11, P3-X63-Ag8.653 (653), P3-X63-Ag8-U1 (P3U1), P3-NS-1 (NS-1), SP2/0-Ag14 (SP2/0), etc. derived from mouse, and 210.RC Y3.Ag1.2.3 (Y3), etc. derived from rat can be used, but it is preferred to use myeloma cells which will not produce by secretion the antibodies out of the cells such as 653, P3U1, NS-1, SP2/0, etc.

### (iv) Cell fusion

Cell fusion can be effected by mixing the lymphocytes of the animal immunized as described above and the myeloma cells at a ratio of cells of (5 to 20) : 1 with the use of a cell suspension which will bring about no trouble to cell fusion, for example, a solution of medium components for culturing lymphocytes generally used (medium components such as MEM, DMEM, McCoy, RPMI1640, etc.), isotonic buffer, etc. and adding to the pellet after centrifugation (cell mass), HVJ (Sendai virus) or PEG (polyethylene glycol) solution, but it is preferred to use a PEG solution, more preferably a 30 to 60 % by weight of PEG solution having an average molecular weight of 1,000 to 8,000. At this time, for promoting cell fusion, corhitin, dimethyl sulfoxide, poly-L-alginine, etc. can be also added.

As the myeloma cell to be used for cell fusion, one derived from an animal different in species from the immunized animal can be also used, but in view of the antibody amount produced and stability of the monoclonal antibody producing hybridoma strain, it is preferred to use a myeloma cell from an animal of the same species as the immunized animal, more preferably of the same strain.

### (v) Selection of hybridoma:

Selection of hybridoma can be performed by culturing the cells after the manipulation of cell fusion in HAT medium (medium containing hypoxanthine, aminopterin, thymidine and fetal bovine serum; as the medium components, medium components for culturing lymphocytes generally employed can be used).

Hybridoma is cultured by placing a number of cells suitable for screening of the antibody producing well in each well (culturing well) of the culturing plate, and at this time a substance for promoting growth of the hybridoma or cells producing the same (e.g. lymphocytes derived from thymus, spleen, lymph node, etc.) can be used as the feeder cell, if desired.

The hybridoma selected by growth in HAT medium is cultured for several days in HT medium (medium containing hypoxanthine, thymidine and fetal bovine serum; as the medium components, medium components for culturing lymphocytes generally employed can be used) until reaching the number of cells suitable for screening of the antibody producing well, and further cultured in a medium for culturing lymphocytes containing fetal bovine serum generally employed.

### (vi) Selection of antibody producing hybridoma:

Assay whether the hybridoma obtained in the above (v) has produced the desired antibody or not can be practiced by, for example, the ELISA method (enzyme immunoassay), the plaque formation method, the agglutination reaction method, RIA (the method by use of radioisotope), but it should be preferably conducted by the ELISA method.

The ELISA method is conducted as described below.

Into each well (assay well) of the ELISA plate having the antigen for analysis prepared in (i) was added the hybridoma culture supernatant, followed by standing for a certain period of time. And, an enzyme-labeled antibody capable of binding through the reaction with the antibody derived from the animal bound to each of these washed assay wells (the enzyme used for labeling may include, for example, peroxidase, alkaline phosphatase, β-galactosidase, etc.; the antibody labeled is not particularly limited, provided that it can be bound through the reac tion with only the antibody derived from the animal bound to the assay well, including, for example, antisera obtained from mouse, rat, rabbit, goat, etc. or monoclonal antibodies produced by the hybridoma strains prepared by use of mouse cells) is added to these assay wells, followed by standing for a certain period of time. Next, these assay wells are washed and the substrate solution corresponding to the enzyme used is added for assay of enzyme activity. And, if enzyme activity is recognized, it can be understood that the hybridoma producing the desired antibody has existed in the culturing well from which the culture supernatant is taken.

Thus, hybridomas exhibiting cell growth and producing antibody can be obtained.

### (vii) Cloning of hybridoma:

The hybridoma in the culturing well in which antibody production is recognized can be subjected to cloning according to the limiting dilution method, the single cell manipulation method (the method in which one hybridoma is introduced under inverted microscope), the method in which the colony is picked up with the use of soft agar, the method by use of FACS (Fluorescent Activated Cell Sorter), etc. At this time, the antibody production hybridoma found in (vi) is cultured according to either one of the cloning methods as mentioned above, and by use of the supernatant in which its growth is recognized, the antibody producing well is screened according to the same method as the ELISA method conducted in selection of the antibody producing hybridoma of (vi).

Thus, a hybridoma strain producing a monoclonal antibody having high specificity for MA and also high antibody value can be selected.

### Method for preparing monoclonal antibody:

The monoclonal antibody having high specificity for MA and high antibody value can be produced by culturing the hybridoma strain obtained in the above (vii) is a flask or culturing it intraperitoneally in an animal.

Production of the hybridoma strain obtained in the above (vii) by culturing in a flask can be performed by culturing it in, for example, a medium for culturing lymphocytes generally employed containing 0 to 20 % fetal bovine serum (e.g. medium containing medium components such as MEM, DMEM, McCoy, RPMI1640, etc.) until the cell concentration reaches the upper limit. At this time, said monoclonal antibody is contained in the culture supernatant obtained by centrifugal operation.

On the other hand, although production of said monoclonal antibody by intraperitoneal culturing of the hybridoma obtained in the above (vii) in animal can be also performed by use of an animal heterologous from the animal from which the cells used for cell fusion are derived, it should be preferably performed by use of a homologous animal species, more preferably an animal of the same strain.

Production of said monoclonal antibody having high specificity for MA and high antibody titer according to such method can be practiced by administering a substance for lowering immune ability of the animal, for example, a mineral oil such as pristan, intraperitoneally into a suitable animal such as mouse, rat, hamster, etc. and several weeks later administering 10⁶ to 10⁷ cells of the hybridoma strain cells obtained in the above (vii) and permitting the strain cells to grow intraperitoneally within several weeks. At this time, said monoclonal antibody is contained in the ascites supernatant obtained by centrifugal operation. The antibody concentration is 10 to 1000-fold of the antibody concentration in the culture supernatant obtained by culturing in a flask.

Said monoclonal antibody obtained by culturing of the hybridoma strain in a flask or intraperitoneally in an animal can be purified by salting out, dialysis, ion exchange chromatography, affinity chromatography, etc. applied for general purification methods of proteins to become a monoclonal antibody of high purity.

The monoclonal antibody obtained as described above has very high specific reactivity with MA.

The MA assay kit to be used in the method for assay MA of the present invention comprises essentially a methamphetamine bound macromolecular protein (hereinafter abbreviated to as "MA bound protein") and a reagent having the monoclonal antibody having very high specific immuno-reactivity with methamphetamine labeled with an enzyme (hereinafter abbreviated to as "enzyme-labeled antibody") or a monoclonal antibody having very specific immunoreactivity for MA (hereinafter abbreviated to as "monoclonal antibody of MA") and an enzyme-labeled MA. For assay of MA, in addition to these reagents, solid phase support, washing solution, blocking solution, MA solution for preparation of calibration curve of MA, substrate solution of enzyme, etc. are also required, and these may be previously assembled in the assay kit of MA, or they may be prepared before assay.

Preparation of the "MA bound protein" in the MA assay kit of the present invention is required for permitting the MA immobilized through a macromolecular protein onto a solid phase support to react with the reagent having the mono clonal antibody having very high specific immunoreactivity with MA labeled with an enzyme. As the macromolecular protein to be used for its preparation, there can be included bovine serum albumin (BSA), ovalbumin (OVA), key-hole limpet hemocyanin (KLH), γ-globulin, etc.

The method for binding MA with the macromolecular protein can be practiced by binding MA with either one of the macromolecular proteins as mentioned above by use of 1-ethyl-3-(3-dimethylaminopropyl)carbodiimide hydrochloride (hereinafter abbreviated to as "EDPC"), 1-cyclohexyl-1-3-(2-morpholinoethyl)carbodiimide metho-p-toluenesulfonate (hereinafter abbreviated to as "CMEC"), etc. Although this can be used as such for assay of MA as the "MA bound protein", for further enhancement of the assay sensitivity of MA, it is preferred to purify this according to gel filtration by use of Sephadex, Sephacryl (both trade names), etc. before use.

The "MA bound protein" fraction obtained by such gel filtration can be also use as such (when the protein concentration is low, it is concentrated through an ultra-filtration membrane, etc. to a desired concentration, while when the protein concentration is high, it is diluted to a desired concentration), but it should be preferably dialyzed with a buffer adjusted to pH around neutral (e.g. phosphate buffer or Tris-hydrochloride buffer), stored after lyophilization or filtration through sterilizing filter, and then used as a "MA bound protein" solution, if desired, at a protein concentration of 0.1 to 100 µg/ml, preferably 1 to 20 µg/ml.

Also, if necessary, a preservative for protein such as sodium azide can be added in a small amount to the "MA bound protein".

In the preparation of the "enzyme-labeled antibody" of the MA assay kit of the present invention,
(viii) the enzyme for labeling the antibody may include at least one selected from oxidoreductase such as peroxidase, catalase, glucose oxidase, lactate oxidase, alcohol oxidase, monoamine oxidase, β-galactosidase, etc., and phosphate hydrolases such as alkaline phosphatase, etc.

As the antibody labeled with the above enzyme, there may be included the monoclonal antibodies produced by the strains MA-7 (FERM-P 1494), MA-13 (FERM-P 1495) and MA-15 (FERM-P 1496) which are hybridoma strains as described above.

The "enzyme-labeled antibody" of the present invention can be prepared by binding at least one monoclonal antibody as mentioned above with at least one enzyme as mentioned above according to the one step method by use of glutaraldehyde [Immunochemistry, vol. 6, p. 43 (1969)] or the two step method [Immunochemistry, vol. 8, p. 1175 (1971)], the periodic acid oxidation method [Method in Enzymology, vol. 37, p. 133 (1975)], etc., of which the latter two methods are preferred.

This can be used as such for assay of MA, but for the purpose of further enhancing assay sensitivity of MA, it is preferred to use the "enzyme-labeled antibody" obtained by purifying this by gel filtration with the use of Sephadex, Sephacryl (both trade names), etc. as the assay reagent for MA.

Although the "enzyme-labeled antibody" obtained by such gel filtration can be also used as such, it should be preferably dialyzed with a buffer adjusted to pH around neutral (e.g. phosphate buffer or Tris-hydrochloride buffer), stored after lyophilization of filtration through sterilizing filter, and then used as an "enzyme-labeled antibody" solution, if desired, at a protein concentration of 0.01 to 100 µg/ml, preferably 0.1 to 10 µg/ml.

The solid phase support to be used in the MA assay method of the present invention is required as the support for immobilizing the "MA bound protein" of the MA assay kit.

The shape of the solid phase support which can be used for immobilizing the MA bound macromolecular protein may include, for example, plate, tube, beads, membrane, etc. for immunoassay, and its material may include, for example, polyethylene, polystyrene, polypropylene, nitrocellulose, glass, etc.

The washing solution to be used in the MA assay of the present invention is required for washing away the "MA bound protein" not immobilized on the solid phase support after immobilization of a certain amount of the "MA bound protein" thereon, or washing away the unreacted substance after the competitive reaction between the MA in the sample to be assayed and the "MA bound protein" immobilized on the solid phase support with the "enzyme-labeled antibody". Also, the washing solution may be used as a diluent for a sample to be assayed.

As the washing solution to be used for such purpose, there can be included, for example, water, a buffer adjusted to the pH during the reaction (buffer such as phosphate buffer, Tris-hydrochloride buffer, etc.), the above buffer containing 0 to 3 % by volume of a surfactant such as Tween 20 and Tween 60 (both trade names), but it is preferred to use a buffer containing 0.02 to 0.8 % by volume of the above surfactant adjusted to the pH during the reaction.

The blocking solution to be used in the MA assay method of the present invention for preventing non-specific binding of the "enzyme-labeled antibody" to the solid phase support having no "MA bound protein" immobilized thereon.

The blocking solution to be used for such purpose can be prepared by dissolving a macromolecular protein such as BSA (bovine serum alubmin), OVA (ovalbumin), KLH (key-hole limpet hemocyanin), γ-globulin, etc. in a washing solution adjusted to the pH during the reaction, and the concentration of these macromolecular proteins may be 0.1 to 10 weight/volume %, preferably 0.1 to 2 weight/volume %, and when it is preferred by use of sera of various animals, the concentration should be made 1 to 50 volume/volume %, preferably 10 to 20 volume/volume %, with the use of the above washing solution.

Also, if necessary, the blocking solution can also be contain a necessary amount of a preservative for protein such as sodium azide, sodium ethylmercurithiosalicylate, etc. added therein.

As the sample to be used in the MA assay method of the present invention, human body fluid such as human urine, blood, serum, etc. can be used.

In the assay of MA, MA is assayed by use of these samples to be assayed diluted with the above washing solution within the range at which MA can be assayed.

As the substrate solution for enzyme to be used in the MA assay method of the present invention (hereinafter abbreviated to as "substrate solution"), a buffer containing the substrate cf H₂O₂ with which the enzyme used for the label of the antibody in the "enzyme-labeled antibody" reacts, and a substance which is colored by the product formed by the enzymatic reaction such as o-phenylenediamine, 2,2′-amino-bis(3-ethylbenzothiazoline-6-sulfonic acid) (ABTS), etc.

As described above, the solid phase support, the washing solution, the blocking solution, the MA solution for preparation of the calibration curve of MA and the "substrate solution" are prepared, and by use of the "MA bound protein", the "enzyme-labeled antibody", which is the assay kit of MA, the MA in a sample to be assayed can be assayed via the respective steps as shown below.

### (1) The step of immobilizing a certain amount of the "MA bound protein" on the solid phase support:

A certain volume of a solution of the "MA bound protein" is brought into contact with the solid phase support for a certain period of time. The temperature during contact is not particularly limited, so long as the "MA bound protein" is not frozen or boiled, but may be preferably 2 to 40 °C.

### (2) The step of removing the "MA bound protein" not immobilized on the solid phase support:

After the "MA bound protein" is brought into contact with the solid phase support for a certain period of time, the "MA bound protein" solution is removed, and further the remaining "MA bound protein" not immobilized on the solid phase support is removed by washing with the washing solution for several times.

### (3) The steps of preventing non-specific binding of the "enzyme-labeled antibody" to the solid phase support surface having no "MA bound protein" immobilized thereon:

A certain volume of the blocking solution is brought into contact with the above solid phase support having the "MA bound protein" immobilized thereon for a certain period of time. The temperature during contact is not particularly limited, so long as the blocking solution is not frozen or boiled, but may be preferably 2 to 40 °C.

### (4) The step of allowing the MA in a sample to be assayed and the "MA bound protein" immobilized on the solid phase support to react competitively with the "enzyme-labeled antibody":

Equal volume of the sample to be assayed and the "enzyme-labeled antibody" solution are allowed to contact and react with the "MA bound protein" immobilized on the solid phase support as described above. The sample to be assayed and the "enzyme-labeled antibody" solution may be either subjected to the contact reaction with the "MA bound protein" immediately after mixing, or alternatively each successively contacted with the "MA bound protein", followed immediately by stirring to effect the reaction.

### (5) The steps of removing the "enzyme-labeled antibody" not immobilized on the solid phase support through the "MA bound protein":

The above mixture of the sample to be assayed and the "enzyme-labeled antibody" solution is removed, and the mixture of the sample to be assayed and the "enzyme-labeled antibody" solution remaining without binding to the solid phase support through the "MA bound protein" is removed by washing with the washing solution for several times.

### (6) The step of allowing the "enzyme-labeled antibody" immobilized on the solid phase support through the "MA bound protein" with the "substrate solution":

As the "substrate solution" to be used here, one corresponding to the enzyme used for labeling of the "enzyme-labeled antibody" (including the substance which is colored when the enzyme reaction occurs) is added.

Its certain volume is allowed to react with the "enzyme-labeled antibody" immobilized on the solid phase support through the "MA bound protein" as described above for a certain period of time, and the enzyme reaction should be preferably stopped with the use of an acid such as H₂SO₄, a base such as NaOH or an enzyme inhibitor. The reaction temperature will pose no problem within the optimum temperature range for the enzyme employed, but may be preferably 20 to 35 °C.

### (7) The step of assay the absorbance of the reaction mixture after the above enzyme reaction:

The absorbance of the reaction is assayed at the wavelength when coloration of the reaction mixture after the above enzyme reaction exhibits the maximum absorbance.

A calibration curve can be prepared from the results obtained by use of the known amounts of MA in place of the sample to be assayed as described above, from which the MA content in the sample to be assayed can be known.

In the step (4) of adding the "enzyme-labeled antibody" solution and the sample to be assayed of the procedure of the MA assay operation, the competitive reactions of the MA of the "MA bound protein" immobilized on the solid phase support and the MA in the sample to be measured with the "enzyme-labeled antibody" occur, whereby the MA content in the sample can be assayed rapidly and at high sensitivity.

As the "monoclonal antibody of MA" of another MA assay kit of the present invention, it can be prepared by use of a monoclonal antibody produced by the strains MA-7 (FERM-P 1494), MA-13 (FERM-P 1495), MA-15 (FERM-P 1496) which are hybridoma strains as described above. Powder or aqueous solution comprising at least one of such monoclonal antibodies can be used as the "monoclonal antibody of MA" of the MA assay kit. When the "monoclonal antibody of MA" to be assayed during MA assay is powder, it may be used by dissolving it in water to produce an aqueous monoclonal antibody solution with an appropriate concentration. When it is an aqueous solution, it may be used by diluting it with an aqueous solution to an appropriate concentration.

In the preparation of the enzyme-labeled MA of the MA assay kit of the present invention, the enzyme for labeling the antibody may include oxidoreductase such as peroxidase, catalase, glucose oxidase, lactate oxidase, alcohol oxidase, monoamine oxidase, β-galactosidase, etc., and phosphate hydrolases such as alkaline phosphatase, etc.

The method for binding MA with enzyme for labeling MA with the above enzyme can be practiced by binding MA with either one of the enzymes as mentioned above by use of EDPC, CMEC, etc.

Although this can be used as such for assay of MA as the enzyme-labeled MA of the assay kit, for further enhancement of the assay sensitivity of MA, it is preferred to purify this according to gel filtration by use of Sephadex, Sephacryl (both trade names), etc. before use.

The enzyme-labeled MA fraction obtained by such gel filtration can be also used as such as the enzyme-labeled MA of the MA assay kit (when the protein concentration is low, it is concentrated through an ultrafiltration membrane, etc. to a desired concentration, while when the protein concentration is high, it is diluted to a desired concentration), but it should be preferably dialyzed with a buffer adjusted pH with high enzyme stability (e.g. phosphate buffer or Tris-hydrochloride buffer), lyophilized or filtered through a sterilization filter to pro vide a MA assay kit. When the enzyme-labeled MA of the lyophilized MA kit is used, it is dissolved in water to be used as an enzyme-labeled MA solution with an aqueous solution, it is diluted with an aqueous solution to an appropriate concentration before use. The enzyme-labeled MA solution at this time should be preferably used at a protein concentration of 2 µg/ml, preferably 0.3 µg/ml.

Also, if necessary, to the enzyme-labeled MA of the MA assay kit can be also added a necessary amount of a preservative for protein such as sodium azide, sodium ethylmercurithiosalicylate, etc., a stabilizing substance for protein such as bovine serum albumin, within the range which will not inhibit enzyme activity.

The solid phase support to be used in the MA assay method of the present invention is required as the support for immobilizing the "monoclonal antibody of MA" of the MA assay kit. The shape of such solid phase support can include, for example, plate, tube, beads, membrane, etc. for immunoassay, and its material can include, for example, polyethylene, polystyrene, polypropylene, nitrocellu lose, glass, etc. In the MA assay method, when the monoclonal antibody having very high specificity for MA immobilized on the solid phase support (maintained under wet state with a solution containing a necessary amount of a preservative for protein such as sodium azide, sodium ethylmercurithiosalicylate, etc., hereinafter abbreviated to as "antibody immobilized support") is used as the MA assay kit in place of the "monoclonal antibody of MA", the time required for assay of MA can be shortened.

The washing solution to be used in the MA assay of the present invention is required for washing away the "monoclonal antibody of MA" not immobilized on the solid phase support after immobilization of a certain amount of the "monoclonal antibody of MA" thereon, or washing away the unreacted substance after the competitive reaction between the MA in the sample to be assayed and the "enzyme-labeled antibody" with the "monoclonal antibody of MA" immobilized on the solid phase support. Also, the washing solution may be used as a diluent for a sample to be assayed.

As the washing solution to be used for such purpose, there can be included, for example, water, a buffer adjusted to the pH during the reaction (buffer such as phosphate buffer, Tris-hydrochloride buffer, etc.), the above buffer containing 0 to 3 % by volume of a surfactant such as Tween 20 and Tween 60 (both trade names), but it is preferred to use a buffer containing 0.02 to 0.8 % by volume of the above surfactant adjusted to the pH during the reaction.

The blocking solution to be used in the MA assay method of the present invention for preventing non-specific binding of the "enzyme-labeled MA" to the solid phase support having no "monoclonal antibody of MA" immobilized thereon.

The blocking solution to be used for such purpose can be prepared by dissolving a macromolecular protein such as BSA, OVA, KLH, γ-globulin, etc. in the above buffer and the concentration of these macromolecular proteins may be 0.1 to 5 weight/volume %, preferably 0.5 to 2 weight/volume %, and when it is preferred by use of sera of various animals, the concentration should be made 1 to 50 volume/volume %, preferably 10 to 20 volume/volume %, with the use of the above washing solution.

Also, if necessary, the blocking solution can also be contain a necessary amount of a preservative for protein as mentioned above added therein.

As the sample to be used in the MA measuring method of the present invention, human body fluid such as human urine, blood, serum, etc. can be used.

In the assay of MA, MA is assayed by use of these samples to be assayed diluted with the above washing solution within the range at which MA can be assayed.

As the "substrate solution" for enzyme to be used in the MA assay method of the present invention, a buffer containing the substrate of H₂O₂ with which the enzyme used for the label of the antibody in the "enzyme-labeled antibody" reacts, and a substance which is colored by the product formed by the enzymatic reaction such as o-phenylenediamine or ABTS.

As described above, the solid phase support, the washing solution, the blocking solution, the MA solution for preparation of the calibration curve of MA and the "substrate solution" are prepared, and by use of the "monoclonal antibody of MA", the "enzyme-labeled antibody", which is the assay kit of MA, the MA in a sample to be assayed can be assayed via the respective steps as shown below.

### (1) The step of immobilizing a certain amount of the "monoclonal antibody of MA" on the solid phase support:

A certain volume of a solution of the "monoclonal antibody of MA" is brought into contact with the solid phase support for a certain period of time. The temperature during contact is not particularly limited, so long as the "monoclonal antibody of MA" is not frozen or boiled, but may be preferably 2 to 40 °C.

### (2) The step of removing the "monoclonal antibody of MA" not immobilized on the solid phase support:

After the "monoclonal antibody of MA" is brought into contact with the solid phase support for a certain period of time, the "monoclonal antibody of MA" solution is removed, and further the remaining "monoclonal antibody of MA" not immobilized on the solid phase support is removed by washing with the washing solution for several times.

### (3) The steps of preventing non-specific binding of the "enzyme-labeled MA" to the solid phase support surface having no "monoclonal antibody of MA" immobilized thereon:

A certain volume of the blocking solution is brought into contact with the above solid phase support having the "monoclonal antibody of MA" immobilized thereon for a certain period of time. The temperature during contact is not particularly limited, so long as the blocking solution is not frozen or boiled, but may be preferably 2 to 40 °C.

### (4) The step of allowing the MA in a sample to be assayed and the "monoclonal antibody of MA" immobilized on the solid phase support to react competitively with the "enzyme-labeled MA":

Equal volume of the sample to be assayed and the "enzyme-labeled MA" solution are allowed to contact and react with the "monoclonal antibody of MA" immobilized on the solid phase support as described above. The sample to be assayed and the "enzyme-labeled MA" solution may be either subjected to the contact reaction with the "monoclonal antibody of MA" immediately after mixing, or alternatively each successively contacted with the "monoclonal antibody of MA", followed immediately by stirring to effect the reaction.

### (5) The steps of removing the "enzyme-labeled MA" not immobilized on the solid phase support through the "monoclonal antibody of MA":

The above mixture of the sample to be assayed and the "enzyme-labeled MA" solution is removed, and the mixture of the sample to be assayed and the "enzyme-labeled MA" solution remaining without binding to the solid phase support through the "monoclonal antibody of MA" is removed by washing with the washing solution for several times.

### (6) The step of allowing the "enzyme-labeled MA" immobilized on the solid phase support through the "monoclonal antibody of MA" with the "substrate solution":

As the "substrate solution" to be used here, one corresponding to the enzyme used for labeling of the "enzyme-labeled MA" (including the substance which is colored when the enzyme reaction occurs) is added.

Its certain volume is allowed to react with the "enzyme-labeled MA" immobilized on the solid phase support through the "monoclonal antibody of MA" as described above for a certain period of time, and the enzyme reaction should be preferably stopped with the use of an acid such as H₂SO₄, a base such as NaOH or an enzyme inhibitor. The reaction temperature will pose no problem within the optimum temperature range for the enzyme employed, but may be preferably 15 to 35 °C.

### (7) The step of assay the absorbance of the reaction mixture after the above enzyme reaction:

The absorbance of the reaction is assayed at the wavelength when coloration of the reaction mixture after the above enzyme reaction exhibits the maximum absorbance.

A calibration curve can be prepared from the results obtained by use of the known amounts of MA in place of the sample to be assayed as described above, from which the MA content in the sample to be assayed can be known.

In the step (4) of adding the "enzyme-labeled MA" solution and the sample to be assayed of the procedure of the MA assay operation, the competitive reactions of the "enzyme-labeled MA" and the MA in the sample to be assay to the "monoclonal antibody of MA" immobilized on the solid phase support occur, whereby the MA content in the sample can be assayed rapidly and at high sensitivity.

The MA assay kit to be used in the method for assay MA of the present invention comprises essentially a carrier having a monoclonal antibody having very high specific immunoreactivity for MA carried thereon (hereinafter abbreviated to as "antibody carrier") and a carrier having MA carried thereon (hereinafter abbreviated to as "MA carrier"). For assay of MA, in addition to these, the reaction plate which becomes the place for these reactions, the known amount of MA solution (hereinafter abbreviated to as "standard MA solution"), stirring rod, etc. are also required, and these may be previously assembled in the assay kit of MA, or they may be also prepared before assay of MA.

As the monoclonal antibody to MA which can be used for preparation of the "antibody carrier" in the MA assay kit in the present invention, there can be employed a monoclonal antibody having very high specificity for MA, for example, one comprising at least one of the monoclonal antibodies produced by the strains MA-7 (FERM-P 1494), MA-13 (FERM-P 1495), MA-15 (FERM-P 1496), etc. which are hybridoma strains as described above can be used.

When the "antibody carrier" to be used during assay of MA in the present invention is powder, it is used as a suspension with an appropriate concentration by dispersing it in water, a buffer (e.g. phosphate buffer, Tris-hydrochloride buffer, etc.), etc. When the "antibody carrier" is a suspension, it is diluted with a solvent to an appropriate concentration before use.

In preparation of the "antibody carrier" or "MA carrier" in the assay kit of the present invention, the material of the carrier for carrying the monoclonal antibody or MA can include polyethylene, polystyrene, polypropylene, nitrocellulose, glass, nylon (trade name), PMMA (polymethyl methacrylate), styrene-divinylbenzene copolymer, styrene-butadiene copolymer, etc., but polystyrene may be preferably used.

These carriers can be used with shapes such as spherical, rod, needle, cubic shapes, etc., but preferably spherical shapes.

These carriers can be used with sizes of 0.1 to 1.5 µm, preferably 0.5 to 1.0 µm, more preferably 0.7 to 0.9 µm. Also, the carriers employed should be preferably uniform.

These carriers can be also appropriately colored, if desired in relationship with the color of the reaction plate used during the assay.

The "antibody carrier" in the present invention can be prepared as described below.

A monoclonal antibody having very high specificity for MA is dissolved in a buffer controlled to around neutral (e.g. phosphate buffer, Tris-hydrochloride) to prepare a solution with an appropriate concentration, which solution is contacted with the above carrier for a certain period of time, whereby the monoclonal antibody can be carried on the carrier. The contact reaction at this time can be carried out preferably under shaking, and also at 20 to 40 °C.

The "antibody carrier" thus obtained can be also stored under the suspension state at a low temperature (e.g. 2 to 10 °C), but preferably a preservative for protein such as sodium azide, sodium ethylmercurithiosalicylate, etc., a stabilizing substance for protein such as bovine serum albumin, etc. can be also added in necessary amounts.

The "MA carrier" in the present invention can be prepared by binding MA and a carrier indirectly through a macromolecular protein.

The macromolecular protein to be used in preparation of the "MA carrier" may include BSA, OVA, KLH, γ-globulin, etc.

Binding of MA and the macromolecular protein in preparation of the "MA carrier" can be practiced by binding MA with the above macromolecular protein by use of EDPC, CMEC, etc.

The "MA carrier" can be obtained by having the bound product of MA and the macromolecular protein thus obtained as such or after purification through gel filtration by use of Sephadex, Sephacryl (both trade names), etc. carried physically or chemically onto a carrier.

The "MA carrier" thus obtained can be also stored under the suspension state at a low temperature (e.g. 2 to 10 °C), but preferably a preservative for protein such as sodium azide, sodium ethylmercurithiosalicylate, etc., a stabilizing substance for protein such as BSA, etc. can be also added in necessary amounts.

As the sample to be assayed in assay of MA of the present invention, human body fluid as mentioned above can be used. And, these samples to be assayed can be used as such without dilution for assay.

As the reaction plate which becomes the place for the reaction to be used in assay of MA in the present invention, there may be included glass plate, plastic plate, paper board coated with a water-proof substance, etc., but it is preferred to use a plate made of paper coated with a plastic material. The reaction plate may have a color which is not limited, so long as the agglutinated substance formed as the result of the reaction between the "MA carrier" and the "antibody carrier" can be clearly confirmed, but, for example, when the agglutinated substance is white, it should be preferably made black.

As the stirring stick for the reaction mixture to be used in assay of MA in the present invention, it is not particularly limited so long as it is water-proof, but prefer ably a plastic stick or a stick coated with a plastic material may be used. The size is not particularly limited, so long as stirring can be effected to achieve the objects of the present invention, but, for example, a stick with a length of 3 to 10 cm and a diameter of 0.1 to 5 mm can be used.

As described above, the reaction plate, the stirring stick for the reaction mixture, the samples to be assayed and the "standard MA solution" are prepared, and by use of the "antibody carrier" and the "MA carrier" which are reagents of the MA assay kit, MA in a sample to be assayed can be assayed via the following steps (i) to (ii).

### (i) The step of placing certain amounts of "antibody carrier", "MA carrier", and sample to be assayed or "standard MA solution":

Each of "antibody carrier", "MA carrier", sample to be assayed or "standard MA solution" is placed each in a certain amount (for example, 5 to 50 µl) as one set with positional intervals on the reaction plate apart from each other so that they can be mutually stirred easily. At this time, it is preferred to place the set comprising ["antibody carrier", "MA carrier" and sample to be assayed] and the set comprising ["antibody carrier", "MA carrier" and "standard MA solution"] at appropriate positional intervals apart from each other on the same reaction plate.

### (ii) The step of allowing the respective solutions on the above reaction plate of (i) to react with each other by stirring each set:

Each solution was quickly stirred with a stirring stick to carry out the reaction while moving the reaction plate, forward and backward, left and right, at a constant temperature (e.g. room temperature) for a certain period of time (e.g. 1 to 2 minutes), and the agglutination of each set formed by the reaction between the "antibody carrier" and the "MA carrier" is observed. At this time, by observing with comparison between the agglutination in the case of the set by use of the "standard MA solution" and the agglutination in the case of the set by use of the sample to be assayed, the MA content in the sample to be assayed can be known.

In the step of carrying out the respective reagents with stirring on the reaction plate for each set of (ii) in the procedure of the MA assay operations, competitive reactions occur between the MA in the "MA carrier" and the MA in the sample to be assayed or the "standard MA solution" relative to the monoclonal antibody of MA carried on the "antibody carrier", and the "antibody carrier" and the "MA carrier" undergo the agglutination reaction, whereby the MA content in the sample can be assayed rapidly and with high sensitivity through observation with comparison between the agglutination in the case of the set by use of the "standard MA solution" and the agglutination in the case of the set by use of the sample to be assayed.

### EXAMPLES

The present invention is described in detail be referring to Examples. These Examples should not be construed as limitative of the present invention.

### Example 1

### [Preparation of immunogen and antigen for analysis]

In 12 ml of benzene, 0.5 mg of methamphetamine (MA) was dissolved, and to the solution were added 1.16 g of N-(4-bromobutyl)phthalimide and 0.54 g of sodium carbonate, followed by reflux under 80 °C for 32 hours. After removal of the inorganics, the mixture was introduced into 12 ml of 1N hydrochloric acid, and the aqueous layer was washed with benzene. Into the aqueous layer was added chloroform, and the chloroform layer obtained was washed with a saturated aqueous sodium chloride solution, dehydrated over anhydrous sodium sulfate, followed by evaporation of chloroform under reduced pressure. The residue was dissolved in 12 ml of ethanol, and to the resultant solution was added 60 µl of 90 % hydrazine hydrate and, after reflux under 78.5 °C for 2 hours, ethanol was evaporated. The residue was dissolved in 12 ml of 1N hydrochloric acid and washed with chloroform. The mixture was adjusted to pH 10 with 3N sodium hydroxide, chloroform was introduced thereinto and the chloroform layer obtained was washed with a saturated aqueous sodium chloride solution, dehydrated over anhydrous sodium sulfate, followed by evaporation of chloroform under reduced pressure. Thus, 0.2 g of N-(4-aminobutyl)methamphetamine (hereinafter abbreviated to as "ABMA") was obtained.

The immunogen was prepared by use of the above ABMA as described below.

After 20 mg of ABMA and 20 mg of an immunoglobulin (human IgG) which is a biological macromolecule were dissolved in 0.4 ml of dimethylformamide, 12 ml of a 10 % 1-ethyl-3-(3-dimethylaminopropyl)carbodiimide hydrochloride (hereinafter abbreviated to as "EDPC") was charged and, after stirred under room temperature for 3 hours, dialyzed against PBS (Dulbecco's phosphate buffered saline), and the non-dialyzed fraction was lyophilized to obtain 4 mg of a bound product of methamphetamine (MA) and human IgG (hereinafter abbreviated to as "MA-1gG") which is "the antigen corresponding to the desired monoclonal antibody" of the immunogen.

The antigen for analysis was prepared as described below.

In the method for preparing the immunogen as described above, 1-cyclohexyl-3-(2-morpholinoethyl)carbodiimide metho-p-toluenesulfonate (CMEC) in place of EDPC and bovine serum albumin (BSA) in place of human IgG, following the same procedure as described above, to obtain 3 mg of a bound product of MA and BSA (hereinafter abbreviated to as "MA-BSA") which is the antigen for analysis.

### Example 2

### [Preparation of a hybridoma strain producing monoclonal antibody to MA]

### (a) Immunization of mouse and preparation of spleen lymphocytes:

One ml of PBS (Dulbecco's phosphate buffered saline) containing 400 µg of the MA-1gG which is the immunogen prepared in Example 1 dissolved therein and 1 ml of Freund's complete adjuvant were thoroughly mixed, and 0.5 ml of the emulsion obtained was administered intraperitoneally into BALB/c mouse (female, 7 weeks old).

Two (2) weeks and 4 weeks after the initial immunization, 0.5 ml of the emulsion prepared in the same manner as described above was administered intraperitoneally into the above mouse.

Further, 2 weeks later, as the final immunization, 0.2 ml of PBS containing 100 µg of the above antigen dissolved therein was administered intravenously via the tail vein of the above mouse.

From the mouse thus immunized, the spleen was removed on the third day after the final immunization, washed with Petri dish containing RPMI1640 medium (containing medium powder for culturing lymphocytes dissolved in distilled water) under ice-cooling, transferred into another Petri dish containing RPMI1640 medium and loosened with a pair of tweezers.

The suspended lymphocytes thus obtained were suspended in RPMI1640 medium, centrifuged (rotational number: 1000 rpm, time: 5 minutes), resuspended in RPMI1640 medium to provide mouse spleen lymphocytes to be used for cell fusion.

### (b) Cell fusion:

4.5 x 10⁷ of 8-azaguanine resistant mouse myeloma cells (X63-Ag, 653;653) at the logarithmic growth phase and 2.7 x 10⁸ of the above mouse spleen lymphocytes were placed in a plastic conical centrifuge tube of 50 ml volume, mixed and then after centrifugation and removal of the supernatant (rotational number: 1400 rpm, time: 6 minutes), the pellet was loosened by tapping the same centrifuge tube.

While the pellet was vigorously shaken, 1 ml of a 50 % PEG 4000 solution (37 °C) was charged over on minute, followed further by vigorous shaking for 1 minute.

While the same centrifuge tube was shaken gently, RPMI1640 medium (37 °C) was added gradually, finally to 10 ml, and the mixture was centrifuged at room temperature (rotational number: 800 rpm, time: 6 minutes), followed by suction removal of the supernatant.

The pellet was loosened by tapping the same centrifuge tube, suspended in 160 ml of HAT medium (RPMI1640 medium containing 1 x 10⁻⁴ M hypoxanthine, 4 x 10⁻⁷ M aminoputerin, 1.6 x 10⁻⁵ M thymidine and 20 % fetal bovine serum), apportioned each in 100 µl into each culturing well of 19 plates of 96-well culture plates, and cultured by use of a CO₂ incubator (5 % CO₂, 95 % air, 37 °C, humidity: 100 %).

### (c) Selection of hybridoma

Over 2 to 4 weeks from the culturing initiation as described above, (b), whether the antibody to MA was contained or not in the culture supernatant in each well of the culture plate in which cell growth was recognized was examined according to the ELISA method shown below.

First, in each analytic well of a 96-well U-bottomed ELISA plate was apportioned each 50 µl of an MA-BSA solution (2 µg/ml, dissolved in 0.05 M carbonate buffer of pH 9.8) which is the antigen for analysis prepared in Example 1, and the mixture was left to stand overnight at 4 °C (by such treatment, MA-BSA was adsorbed onto the surface of each analytic well).

Subsequently, after each analytic well was washed with a washing solution (PBS containing 0.05 % of Tween 20), each apportioned in each analytic well, left to stand at room temperature for 2 hours, these respective analytic wells were washed with the washing solution, and the culture supernatant in each culture well of the above culture plate was apportioned each in 50 µl into these respective analytic wells and left to stand at room temperature for 2 hours (for the negative control, a supernatant obtained by culturing similarly a mixture of mouse spleen lymphocytes and mouse myeloma cells before fusion was employed; on the other hand, for the positive control, the serum of mouse used for cell fusion in the present invention diluted to 100-fold with the washing liquor was used).

Next, each analytic well in the ELISA plate was washed, and each 50 µl of an alkaline phosphatase labeled antibody solution to mouse immunoglobulin was apportioned into each analytic well and left to stand at room temperature for 1 hour. And, after each analytic well in the ELISA plate was washed, each 100 µl of a sodium p-nitrophenylphosphate 6H₂O solution (1 mg/ml) was apportioned into each analytic well and, after the reaction at room temperature for 30 minutes, absorbance of each well at 405 nm was assayed by means of a spectrophotometer for microplate.

As the result of such investigation, production of antibody to MA was recognized in 34 of 1411 wells in the culture plates.

For the 34 wells having produced these antibodies, inhibition test by use of MA (in the above ELISA, the same operation is performed except for introducing a solution containing 10 µg per well of MA in the supernatant in place of the supernatant in the culture well) was conducted.

As the result, the culture wells containing antibodies inhibited with MA were found in 19 of the 34 wells. Thus, in the 13 culture wells, a hybridoma producing an antibody reactive with MA was confirmed to exist.

### (d) Cloning of hybridoma:

By use of an RPMI1640 medium containing 20 % fetal bovine serum, the hybridoma was cloned according to the single cell manipulation method (the method in which one hybridoma is placed in one well under inverted microscope) for the 3 wells of the 19 culture wells in which antibody production was confirmed as shown in the step (c) as described above.

For culturing, a 96-well culture plate was used, and with the use of BALB/c mouse thymus cell suspension (10⁷ cells/ml) as the feeder cell, culturing was conducted at (one hybridoma)/(100 µl of the thymus cell suspension)/well.

In the above culturing, the supernatant in the culture well where a single colony was observed after around the tenth day was sampled, and screening of the antibody producing well was performed by the ELISA method (the same method as in the above (c)) to obtain 3 hybridoma strains, which were then subjected to recloning.

The strains thus obtained are designated as MA-7 strain (FERM-P 1494), MA-13 strain (FERM-P 1495) and MA-15 strain (FERM-P 1496), and the monoclonal antibodies produced by these strains as MA-7, MA-13 and MA-15.

The class and subclass, the type of L-chain of the monoclonal antibodies contained in the culture supernatants of these 3 strains were determined in the assay test I shown below, and the reactivity for various components in the assay test II.

### Assay test I

### (Determination of class and subclass of monoclonal antibody to MA)

Determination of class and subclass of immunoglobulin produced by MA-7, MA-13 and MA-15 strains was conducted by the same ELISA method as in the step (c) as described above by use of peroxidase-labeled antibody solutions specific for the respective class and subclass of mouse antibodies (antibodies labeled with horseradish peroxidase to IgG₁, IgG₂ₐ, IgG_{2b}, IgG₃, IgM, IgA, κ type L-chain or λ type L-chain, etc.), and by the Ouchterlony method by use of antibody solutions specific for the respective class and subclass of mouse antibodies (antibodies to IgG₁, IgG₂ₐ, IgG_{2b}, IgG₃, IgM, IgA, κ type L-chain or λ type L-chain).

As the result, all of the monoclonal antibodies produced by MA-7 and MA-13 strains (MA-7 and MA-13) were found to be antibodies belonging to IgG₁ having λ type L-chain, and the monoclonal antibody produced by the MA-15 strain (MA-15) to be an antibody belonging to IgG_{2b} having κ type L-chain.

### Assay test II

### (Investigation of reactivities of monoclonal antibodies to MA with various compounds)

In order to determine the reaction specificities of the monoclonal antibodies such as MA-7, MA-13 and MA-15, reactivities with compounds different from MA such as AP (amphetamine), EP (ephedrin), ME (methylephedrin), MPA (methoxyphenamine), PT (phentermine), norEp (norephedrin), DBED (N,N′-dibenzylethylenediamine), OH-MA (p-hydroxymethamphetamine), OH-AP (p-hydroxyamphetamine), OH-EP (p-hydr oxyephedrin), OH-norEP (p-hydroxynorephedrin), Mes (mescaline), etc. were investigated according to the same ELISA method as described above in the step (c) (however, the reagent used for assay was made two-fold in amount, and a mixed solution of 50 µl of a solution of a compound diluted at multiple stages with the washing liquor and 50 µl of a monoclonal antibody solution was used in place of the hybridoma culture supernatant).

The reactivities of these antibodies with MA, AP, EP, ME, MPA, PT, norEp, DBED, OH-MA, OH-AP, OH-EP, OH-norEP and Mes are shown in Table 1 in terms of the reactive ratios relative to MA.

**Table 1**

| Compound | MA-7 | MA-13 | MA-15 |
|---|---|---|---|
| MA | 1 | 1 | 1 |
| AP | 0.0051 | 0.0062 | 0.019 |
| EP | 0.0018 | 0.0025 | 0.0012 |
| ME | 0.022 | 0.028 | 0.016 |
| MPA | 0.023 | 0.0037 | 0.0016 |
| PT | 0.042 | 0.12 | 0.0039 |
| norEP | <0.001 | <0.001 | <0.001 |
| DBED | 0.17 | 0.23 | 0.0055 |
| OH-MA | 0.0039 | 0.14 | 0.014 |
| OH-AP | <0.001 | 0.003 | <0.001 |
| OH-EP | <0.001 | <0.001 | 0.0031 |
| OH-norEP | <0.001 | <0.001 | <0.001 |
| Mes | <0.00001 | <0.00001 | <0.00001 |

### Example 3

### [Production of monoclonal antibody to MA by flask culturing]

The cultured cells of the MA-15 strain obtained by culturing in RPMI1640 medium containing 15 % fetal bovine serum was transferred into 10 ml of RPMI1640 medium (containing no fetal bovine serum) and cultured immediately before death.

The monoclonal antibody to MA (MA-15) was found to be contained at 40 µg/ml (assayed by the single radial immunodiffusion method) in the supernatant obtained by centrifugation (rotational number: 3000 rpm, time: 5 minutes) or the culture broth.

### Example 4

### [Production of monoclonal antibody to MA in mouse abdominal cavity]

For obtaining a large amount of monoclonal antibody to MA, the cells of MA-15 strain were cultured in mouse abdominal cavity.

2 x 10⁶ cells of MA-15 strain suspended in RPMI1640 were administered intraperitoneally into BALB/c mouse (female, 6 weeks old, administered with 0.5 ml of pristan 2 weeks before).

The body weight of the mouse exhibited remarkable gain after about one week, and the ascites (10 ml/mouse) were collected on the second week. The ascites were centrifuged (rotational number: 3000 rpm, time: 5 minutes) to obtain ascitic supernatant.

The monoclonal antibody to MA (MA-15) was found to be contained in an amount of 8.3 mg/ml (assayed by single radial immunodiffusion method) in the ascitic supernatant.

### Reference example 1

### Preparation of "MA bound protein"

Similarly as in Example 1, 0.2 g of ABMA was obtained.

The "MA bound protein" was prepared by use of the above ABMA as described below.

After 0.4 ml of dimethylformamide was mixed with 20 mg of ABMA and an aqueous solution of bovine serum albumin (BSA) (20 mg/ml), 1 ml of 10 % EDPC was added to adjust pH to 5.5 and after stirred at room temperature under light shielding, the mixture was dialyzed against pure water, followed by lyophilization of the non-dialyzed fraction, to obtain 22 mg of a bound product of MA and BSA (hereinafter abbreviated to as "MA-BSA").

### Reference example 2

### Production and purification of antibody

Production of a monoclonal antibody having high specificity for the MA possessed by the present inventors prepared according to the method as described above was performed as described below.

10⁷ strain cells suspended in PBS were administered intraperitoneally into BALB/c mouse (male, 8 weeks old, administered intraperitoneally with 0.5 ml of pristan 2 weeks before). Marked increase of mouse body weight was recognized after about one week, and ascites were suitably taken out after one to 3 weeks. The monoclonal antibody thus obtained has an antibody titer of 10⁶ to 10⁸.

Purification of the monoclonal antibody from the ascites obtained was conducted as follows.

The above ascites were dialyzed against Tris-buffer (pH 7.4), and flowed through a DEAE-cellulose column equilibrated with the same buffer. The fraction passed was salted out with 50 % saturated ammonium sulfate, and the resultant precipitate was dissolved in PBS and dialyzed against the same buffer. The purity of the monoclonal antibody to MA thus obtained was found to be higher than any antibody according to SDs-polyacrylamide gel electrophoresis.

According to the method for assay MA by use of the purified monoclonal antibody to MA obtained as described above (ELISA method), it has become possible to perform high sensitivity and rapid assay of MA.

### Example 5

### Preparation of "MA bound protein" and "enzyme-labeled antibody" for MA assay kit

The "MA bound protein" and "enzyme-labeled antibody" for MA assay kit were prepared as described below.

The "MA bound protein" obtained as shown in Reference example 1 was dialyzed under 4 °C overnight against PBS, apportioned each in 0.2 ml into bottles and stored after lyophilization (5 to 100 µg/bottle) to provide the "MA bound protein" which is the assay kit of the present invention. During assay of MA, 0.2 ml of distilled water was added into this bottle to dissolve well the lyophilized powder, and diluted with PBS to a desired concentration before use.

The antibody in the "enzyme-labeled antibody" was obtained as the purified monoclonal antibody as shown in Reference example 2 by use of the MA-15 strain (FERM-P 1496).

By use of the monoclonal antibody, the antibody was labeled with an enzyme according to the known method as shown below to prepare the "enzyme-labeled antibody" for the assay kit of the present invention.

First, 7.32 g of horseradish peroxidase was dissolved in 1 ml of distilled water and 200 µl of 0.1 M sodium periodate was added, followed by standing at room temperature for 30 minutes. After the enzyme solution was dialyzed at 4 °C overnight against 1 mM acetate buffer (pH 4.5), 100 µl of 0.2 M sodium carbonate buffer (pH 9.5) was added to adjust pH to 9.5. On the other hand, 8 mg of the monoclonal antibody (MA-15) dissolved in PBS was dialyzed at 4 °C overnight against 0.01 M sodium carbonate buffer (pH 9.5). The peroxidase and the monoclonal antibody thus obtained was mixed, left to stand at room temperature for 2 hours and a half, and sodium borohydride was added to the reaction mixture, followed by standing at 4 °C for 2 hours. The peroxidase-labeled monoclonal antibody thus obtained was dialyzed at 4 °C overnight against PBS, and this was diluted to 100-fold with PBS containing 0.01 % sodium ethylmercurithiosalicylate, apportioned each in 0.2 ml into a bottle and stored under lyophilization to provide the "enzyme-labeled antibody" (10 µg/bottle) which is the assay kit of MA. During assay of MA, 0.2 ml of distilled water was added into the bottle to dissolve well the lyophilized powder, and the solution was suitable diluted with PBS before use.

### Example 6

### Preparation of calibration curve by use of "enzyme-labeled antibody"

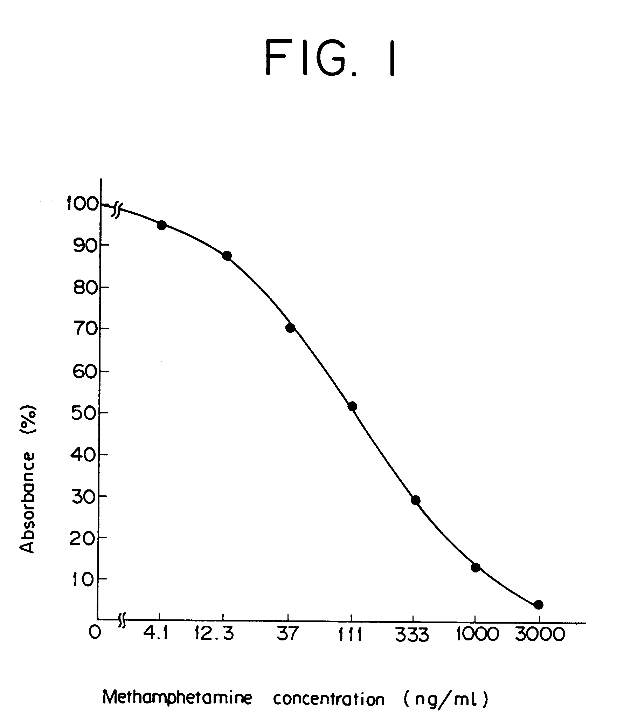

The MA-BSA which is the "MA bound protein" (bound product of MA and BSA, 5 µg/ml) was apportioned each in 100 µl into a 96-well flat-bottomed plate for immunoassay which is the solid phase support (microplate made of polystyrene produced by Nunc Co.), and left to stand under 4 °C overnight to immobilize MA-BSA onto each well. Next, for preventing non-specific adsorption of the "enzyme-labeled antibody" onto the plate, a washing solution containing 10 % calf serum (phosphate buffer of pH 7.4 containing 0.05 % of Tween 20) was placed and left to stand under room temperature for 30 minutes. Next, after washing with the same washing solution, 50 µl of the standard MA solution prepared by using the diluted urine of healthy man (produced by HYLAND DIAGNOSTIC CO.) to 5-fold with the same washing solution [(0 to 150 ng)/50 µl] and 50 µl of the "enzyme-labeled antibody" prepared in Example 1 were added at the same time into each well, followed by mixing and standing under room temperature for 30 minutes. Next, after washed with the same washing solution, the "substrate solution" (20 mg of o-phenylenediamine and 10 µl of 35 % H₂O₂ dissolved in 25 ml of 0.1 M citrate buffer, pH 5.0) was apportioned each in 100 µl and left to stand under light shielding at room temperature for 5 minutes. Finally, each 50 µl of 2N sulfuric acid was apportioned to stop the enzymatic reaction, and absorbance of the solution after stopping of the reaction at 492 nm was assayed by use of a microplate photometer. As the result, the assay method of MA at high sensitivity in about 40 minutes by use of the 96-well flat-bottomed plate for immunoassay having immobilized the "MA bound protein" (microplate made of polystyrene of Nunc Co.). The results are shown in Fig. 1.

### Example 7

### Recovery test of MA

MA was assayed in the same manner as in Example 6 except for using the "solution i.e. MA added urine of healthy man which was diluted 5-fold with washing solution (solution in which MA was added to 30 ng/ml)" in place of the "standard MA solution" in Example 6. As the result, the MA amount added and the MA amount assayed were found to be substantially identical. The results are shown in Table 2.

### Example 8

### Recovery test of MA

MA was assayed in the same manner as in Example 6 except for using the "solution i.e. MA added urine of healthy man which was diluted 5-fold with washing solution (solution in which MA was added to 100 ng/ml)" in place of the "standard MA solution" in Example 6. As the result, the MA amount added and the MA amount assayed were found to be substantially identical. The results are shown in Table 2.

### Example 9

### Recovery test of MA

MA was assayed in the same manner as in Example 6 except for using the "solution i.e. MA added urine of healthy man which was diluted 5-fold with washing solution (solution in which MA was added to 300 ng/ml)" in place of the "standard MA solution" in Example 6. As the result, the MA amount added and the MA amount assayed were found to be substantially identical. The results are shown in Table 2.

### Example 10

### Recovery test of MA

MA was assayed in the same manner as in Example 6 except for using the "solution i.e. MA added urine of healthy man which was diluted 5-fold with washing solution (solution in which MA was added to 1000 ng/ml)" in place of the "standard MA solution" in Example 6. As the result, the MA amount added and the MA amount assayed were found to be substantially identical. The results are shown in Table 2.

### Example 11

### Recovery test of MA

MA was assayed in the same manner as in Example 6 except for using the "solution i.e. MA added urine of healthy man which was diluted 5-fold with washing solution (solution in which MA was added to 3000 ng/ml)" in place of the "standard MA solution" in Example 6. As the result, the MA amount added and the MA amount assayed were found to be substantially identical. The results are shown in Table 2.

**Table 2**

| Example | MA concentration in urine after MA added (ng/ml) | Found value (ng/ml) | Recovery (%) |
|---|---|---|---|
| | 0 | 0 | - |
| 3 | 30 | 36.5 | 121.7 |
| 4 | 100 | 100 | 100 |
| 5 | 300 | 300 | 100 |
| 6 | 1000 | 925 | 92.5 |
| 7 | 3000 | 2650 | 88.3 |

### Example 12

### Recovery test of MA in the presence of methylephedrine

MA was assayed in the same manner as in Example 6 except for using the "solution i.e. MA and methylephedrine (hereinafter abbreviated to as "ME") added urine of healthy man which was diluted 5-fold with washing solution (solution in which ME was added at 0.1, 10 and 100 µg/ml was respectively added into 0.5 µg/ml of MA solution)" in place of the "standard MA solution" in Example 6. As the result, the MA amount added (0.5 µg/ml) and the amount of MA practically assayed (0.35 to 0.55 µg/ml) were found to be substantially identical. The results are shown in Table 3..

### Example 13

### Recovery test of MA in the presence of methylephedrine

MA was assayed in the same manner as in Example 6 except for using the "solution i.e. MA and ME added urine of healthy man which was diluted 5-fold with washing solution (solution in which ME was added at 0.1, 10 and 100 µg/ml was respectively added into 1.0 µg/ml of MA solution)" in place of the "standard MA solution" in Example 6. As the result, the MA amount added (1.0 µg/ml) and the amount of MA practically assayed (1.22 to 1.38 µg/ml) were found to be substantially identical. The results are shown in Table 3.

### Example 14

### Recovery test of MA in the presence of methylephedrine

MA was assayed in the same manner as in Example 6 except for using the "solution i.e. MA and ME added urine of healthy man which was diluted 5-fold with washing solution (solution in which ME was added at 0.1, 10 and 100 µg/ml was respectively added into 5.0 µg/ml of MA solution)" in place of the "standard MA solution" in Example 6. As the result, the MA amount added (5.0 µg/ml) and the amount of MA practically assayed (5.37 to 5.78 µg/ml) were found to be substantially identical. The results are shown in Table 3.

**Table 3**

| Example | Amount of MA added (µg/ml) | Amount of ME added (µg/ml) | | | |
|---|---|---|---|---|---|
| | | 0 | 1 | 10 | 100 |
| Control | 0 | 0.03 | 0.03 | 0.12 | 1.25 |
| 8 | 0.5 | 0.56 | 0.58 | 0.62 | 1.60 |
| 9 | 1.0 | 1.25 | 1.25 | 1.50 | 2.60 |
| 10 | 5.0 | 5.40 | 5.50 | 5.90 | 6.00 |
| Comparative ex. | 5.0 | - | - | 7.00 | 25.00 |
| Comparative example is the value determined from the cross-reactive ratio when assayed with the use of a serum having high cross-reactivity with ME as described in S. Tokura: Anal. Biochem., vol. 161, p. 117 (1987). | | | | | |

### Example 15

### Investigation of reactivity to MA analogous compound

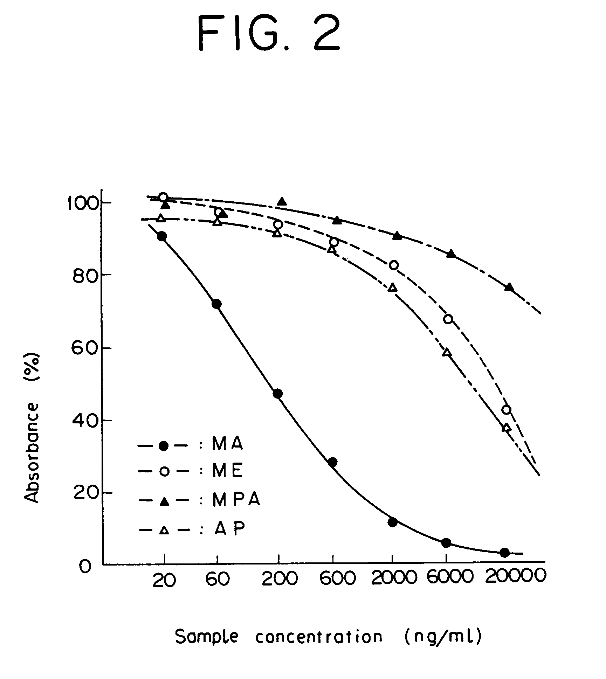

According to Example 6, other than MA, reactivities of MA analogous compounds such as amphetamine (AP), methylephedrine (ME), methoxyphenamine (MP), etc. were assayed. The results are shown in Table 4 and Fig. 2.

**Table 4**

| | | | | |
|---|---|---|---|---|
| Compound | MA | AP | ME | MPA |
| Reactivity (%) | 100 | 1.8 | 1.2 | <0.2 |

### Reference example 3

### Preparation of enzyme-labeled MA

Similarly as in Example 1, ABMA was obtained.

The enzyme-labeled MA was prepared as described below by use of the above ABMA.

A dimethylformamide solution (0.2 ml) containing 10 mg of ABMA [N-(4-aminobutyl)methamphetamine] dissolved therein and 1.5 ml of PBS were mixed, and the solution was dialyzed against 10 mM carbonate buffer (pH 9.5) at 4 °C overnight. On the other hand, 20 mg of horseradish peroxidase was dissolved in 1 ml of pure water, 600 µl of 0.1 M sodium periodate was added thereto and the mixture was left to stand at room temperature for 30 minutes. The enzyme solution was dialyzed against 1 mM acetate buffer (pH 4.5) at 4 °C overnight, and 100 µl of 0.2 M carbonate buffer (pH 9.5) was added to adjust the pH to 9.5. The ABMA solution and the horseradish peroxidase solution thus obtained were mixed, and the mixture was left to stand under room temperature for about 2 hours and a half, followed by addition of sodium borohydride to carry out the reaction at 4 °C for 2 hours. The MA labeled with the enzyme obtained was dialyzed against PBS at 4 °C overnight, stored as such or under lyophilization as the enzyme-labeled MA, and thereafter formed into an enzyme-labeled MA solution before assay of MA.

### Example 16

The enzyme-labeled MA and the "monoclonal antibody to MA" of the MA assay kit were prepared as described below.

The enzyme-labeled MA as obtained in Reference example 3 was dialyzed against PBS at 4 °C overnight, diluted to 100-fold with 0.1 M phosphate buffer (pH .4) containing 0.01 % sodium ethylmercurithiosalicylate, apportioned each in 0.2 ml in bottle and stored under lyophilization to provide the enzyme-labeled MA (10 µg/bottle) which is the MA assay kit. During assay of MA, 0.2 ml of distilled water was added into the bottle to dissolve well the lyophilized powder and diluted with PBS before use.

The "monoclonal antibody to MA" was prepared as shown in Reference example 2. To the monoclonal antibody thus obtained (protein concentration was 250 µg/ml) was added sodium azide to the final concentration of 0.1 % (weight/volume), and the mixture was apportioned each in 0.2 ml in bottle, followed by storage at 4 °C to provide the "monoclonal antibody of MA" which is the assay kit of MA. During assay of MA, this was diluted appropriately with Tris-hydrochloride buffer (pH 7.4) before use.

### Example 17

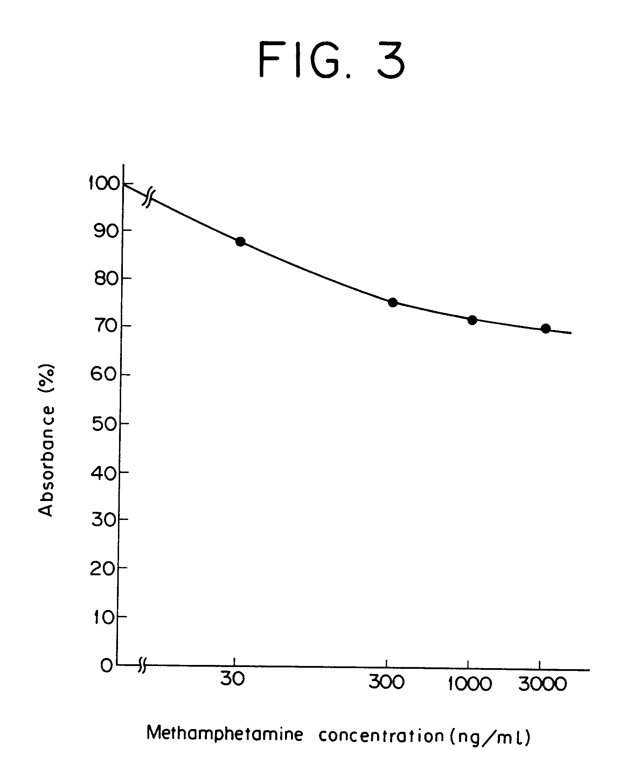

The "monoclonal antibody" (protein concentration was made 5 µg/ml with Tris-hydrochloride buffer (pH 7.4) which was the assay kit of MA prepared in Example 16 was apportioned each in 100 µl in a 96-well flat-bottomed plate for immunoassay (microplate made of polystyrene, produced by Nunc Co.) and left to stand at 4 °C overnight to immobilize the "monoclonal antibody of MA" in each well. Next, for preventing non-specific adsorption of the enzyme-labeled MA onto the plate, a washing solution (phosphate buffer of 0.1 M, pH 7.4 containing 0.05 % by volume of Tween 20) containing 10 % by volume of bovine serum was placed and left to stand at room temperature for 30 minutes. Next, after washing with the same washing solution, 50 µl of the standard MA solution [(0 to 150 ng)/50 µl] prepared by using the diluted the urine of a healthy man with the same washing solution (pH 7.4) to 5-fold as a duluent and 50 µl of the enzyme-labeled MA which was the assay kit prepared in Example 16 dissolved in 0.2 ml of distilled water were added at the same time in each well, followed by standing at room temperature for 30 minutes. Next, after washed with the same washing solution, the "substrate solution" (20 mg of o-phenylenediamine which is the coloring substance and 10 µl of 35 % H₂O₂ which is the substrate dissolved in 0.1 M citrate buffer of pH 5.0) was apportioned each in 100 µl, and left to stand under light shielding at room temperature for 50 minutes. Finally, each 50 µl of 2N sulfuric acid was apportioned to stop the enzymatic reaction, and absorbance of the solution after stopping of the reaction at 492 nm was assayed by use of a microplate photometer. As the result, the assay method of MA by use of the enzyme-labeled MA could assay MA at high sensitivity in about one hour by use of the 96-well flat-bottomed plate (microplate made of polystyrene of Nunc Co.) having the "monoclonal antibody of MA" immobilized thereon. The results are shown in Fig. 3

### Example 18

### [Preparation of "MA carrier"]

In the same manner as in Example 1, AMBA was obtained.

The bound product of MA and BSA which is a macromolecular protein (MA-BSA) in preparation of the "MA carrier" was prepared as described below by use of the above ABMA.

In 3 ml of distilled water was dissolved 20 mg of BSA, and the solution was mixed with 20 mg of ABMA (30 mg/ml dimethylformamide), and then the pH was adjusted to 5.5 by introducing 10 % EDPC. After the mixture was stirred at room temperature under light shielding, it was dialyzed a=ainst pure water and the non-dialyzed fraction was lyophilized to give 22 mg of MA-BSA.

Carrying of MA on the carrier through BSA of MA-BSA in preparation of the "MA carrier" was performed as described below.

A mixture of 100 µl of MA-BSA solution (400 µg/ml) with 100 µl of a polystyrene latex which is the carrier (solid components 4.0 % by weight, particle size 0.8 µm, solvent PBS was shaken at 37 °C for 3 hours to have the MA-BSA carried physically on the polystyrene latex surface to obtain the "MA carrier".

### Example 19

### [Preparation of "antibody carrier"]

The "antibody carrier" was prepared by mixing 100 µl of a monoclonal antibody solution exhibiting very high specificity to the MA obtained as shown in Reference example 2 (2 mg/ml, solvent was 0.1 M phosphate buffer of pH 7.4) and 100 µl of a polystyrene latex (solid components 4.0 % by weight, particle size 0.8 µm, solvent PBS, shaking the mixture at 37 °C for 3 hours to have the monoclonal antibody physically carried on the polystyrene latex surface, thereby obtaining the "antibody carrier".

### Example 20

### [Assay of MA diluted with urine of healthy man]

The "MA carrier" prepared in Example 18 and the "antibody carrier " prepared in Example 19 were added dropwise each in 25 µl separately from each other onto the three sheets of reaction plates, and on the same plates were further added each 5 µl of the samples to be assayed with MA concentrations of 0.5 ppm and 1.0 ppm prepared by dilution with urine of healthy man (produced by HYLAND DIAGNOSTIC CO.) separately from each other, followed immediately by mixing of the "MA carrier", the "antibody carrier" and the sample to be assayed with a stirring stick. When the same plates were continued to be moved slowly, when the MA concentration in the sample to be assayed was 0 ppm, the latex particles were observed with naked eyes within about 30 seconds, while when it was 0.5 ppm, within about 1 minutes and 30 seconds, and when it was 1.0 ppm, within about 4 minutes and 30 seconds.

### Comparative example 1

### [Assay of methylephedrine]

In Example 20, samples to be assayed were prepared by use of methylephedrine (ME) in place of MA and the assay was carried out, following otherwise the same procedure.

As the result of assay, particles of polystyrene latex were observed with naked eyes to be agglutinated within about 30 seconds in either case of the ME concentration of 0 ppm, 0.5 ppm and 1.0 ppm.

### Comparative 2

### [Assay of ephedrine]

In Example 20, samples to be assayed were prepared by use of ephedrine (EP) in place of MA and the assay was carried out, following otherwise the same procedure.

As the result of assay, particles of polystyrene latex were observed with naked eyes to be agglutinated within about 30 seconds in either case of the EP concentration of 0 ppm, 0.5 ppm and 1.0 ppm.

The "monoclonal antibody having very high specificity for methamphetamine (MA) which is an antihypnotic obtained by culturing of a cell strain" of the present invention can be utilized for examination and assay of MA.

Also, according to the present invention, by the use of a assay kit of MA comprising essentially the "MA bound protein" and the "enzyme-labeled antibody", MA can be assayed rapidly and at high sensitivity under the competitive reaction conditions.

Further, according to the present invention, by the use of a assay kit of MA comprising essentially the "monoclonal antibody to MA" and the enzyme-labeled MA, MA can be assayed rapidly and at high sensitivity under the competitive reaction conditions.

Moreover, according to the present invention, by use of a assay kit of MA comprising essentially the "MA carrier" and the "antibody carrier", MA can be assayed with high specificity, rapidity, and high sensitivity and with ease.

## Claims

1. Monoclonal antibodies to methamphetamine obtainable from the hybridoma strains deposited at the Fermentation Research Institute, Agency of Industrial Science and Technology and having the accession numbers FERM BP-1494, FERM BP-1495 or FERM BP-1496.

2. A monoclonal antibody to methamphetamine characterised by the following cross reactivities expressed in terms of reactive ratios relative to methamphetamine:-
methamphetamine 1, amphetamine 0.0051, ephedrine 0.0018, methylephedrine 0.022, methoxyphenamine 0.023, phentermine 0.042, norephedrine <0.001, N,N'-dibenzylethylenediamine 0.17, p-hydroxymethamphetamine 0.0039, p-hydroxyamphetamine <0.001, p-hydroxyephedrine <0.001, p-hydroxynorephedrine <0.001, mescaline <0.00001.

3. A monoclonal antibody to methamphetamine characterised by the following cross reactivities expressed in terms of reactive ratios relative to methamphetamine:-
methamphetamine 1, amphetamine 0.0062, ephedrine 0.0025, methylephedrine 0.028, methoxyphenamine 0.0037, phentermine 0.12, norephedrine <0.001, N,N'-dibenzylethylenediamine 0.23, p-hydroxymethamphetamine 0.14, p-hydroxyamphetamine 0.003, p-hydroxephedrine <0.001, p-hydroxynorephedrine <0.001, mescaline <0.00001.

4. A monoclonal antibody to methamphetamine characterised by the following cross reactivities expressed in terms of reactive ratios relative to methampthetamine:-
methamphetamine 1, amphetamine 0.019, ephedrine 0.0012, methylephedrine 0.016, methoxyphenamine 0.0016, phentermine 0.0039, norephedrine <0.001, N,N'-dibenylethylenediamine 0.0055, p-hydroxymethamphetamine 0.014, p-hydroxyamphetamine <0.001, p-hydroxyephedrine 0.0031, p-hydroxynorephedrine <0.001, mescaline <0.0001.

5. A monoclonal antibody to methamphetamine as claimed in any one of Claims 1 to 4 characterized in that the monoclonal antibody is labelled with at least one enzyme.

6. A monoclonal antibody to methamphetamine as claimed in Claim 5 characterised in that the enzyme is selected from oxidoreductase, peroxidase, catalase, glucose oxidase, lactate oxidase, alcohol oxidase, monoamine oxidase, β-galactosidase, and alkaline phosphatase.

7. A method for assay of methamphetamine characterised by the step of allowing a methamphetamine-bound macromolecular protein immobilized on a solid phase support and methamphetamine in a sample to be assayed, to react competitively with a reagent comprising an enzyme labelled monoclonal antibody according to Claims 5 or 6.

8. A methamphetamine assay kit for use in the method described in Claim 7, comprising;
(a) a methamphetamine-bound macromolecular protein; and
(b) a reagent comprising an enzyme labelled monoclonal antibody according to Claims 5 or 6.

9. A method for assay of methamphetamine characterised by the steps of immobilizing a monoclonal antibody according to any one of Claims 1 to 4, on a solid phase support and allowing enzyme labelled methamphetamine and methamphetamine in a sample to be assayed, to react competitively with the monoclonal antibody immobilized on the solid phase support.

10. A methamphetamine assay kit for use in the method according to Claim 9, comprising;
(a) a monoclonal antibody according to any one of Claims 1 to 4; and
(b) enzyme-labelled methamphetamine.

11. A methamphetamine assay kit comprising:
(a) a carrier having a monoclonal antibody according to any one of Claims 1 to 4 carried thereon; and
(b) a carrier having methamphetamine carried thereon.

12. A method for assay of methamphetamine characterised by the use of a kit as claimed in Claim 11 and comprising the steps of allowing methamphetamine in a sample to be assayed to react competitively with the monoclonal antibody carried on the carrier.

## Patentansprüche

1. Monoklonale Antikörper gegen Methamphetamin, erhältlich von den Hybzidoma-Rassen, die niedergelegt sind bei dem Fermentation Research Institute, Agency of Industrial Science and Technology und welche die Zugriffnummern FERM BP-1494, FERM BP-1495 oder FERM BP-1496 haben.

2. Monoklonaler Antikörper gegen Methamphetamin, gekennzeichnet durch die folgenden Querreaktivitäten, ausgedrückt als reaktive Verhältnisse relativ zu Methamphetamin:
Methamphetamin 1, Amphetamin 0.0051, Ephedrin 0.0018, Methylephedrin 0.022, Methoxyphenamin 0.023, Phentermin 0.042, Norephedrin <0.001, N,N'-Dibenzylethylendiamin 0.17, p-Hydroxymethamphetamin 0.0039, p-Hydroxyamphetamin <0.001, p-Hydroxyephedrin <0.001, p-Hydroxynorephedrin <0.001, Meskalin <0.00001.

3. Monoklonaler Antikörper gegen Methamphetamin, gekennzeichnet durch die folgenden Querreaktivitäten, ausgedrückt als reaktive Verhältnisse relativ zu Methamphetamin:
Methamphetamin 1, Amphetamin 0.0062, Ephedrin 0.0025, Methylephedrin 0.028, Methoxyphenamin 0.0037, Phentermin 0.12, Norephedrin <0.001, N,N'-Dibenzylethylendiamin 0.23, p-Hydroxymethamphetamin 0.14, p-Hydroxyamphetamin 0.003, p-Hydroxyephedrin <0.001, p-Hydroxynorephedrin <0.001, Meskalin <0.00001.

4. Monoklonaler Antikörper gegen Methamphetamin, gekennzeichnet durch die folgenden Querreaktivitäten, ausgedrückt als reaktive Verhältnisse relativ zu Methamphetamin:
Methamphetamin 1, Amphetamin 0.019, Ephedrin 0.0012, Methylephedrin 0.016, Methoxyphenamin 0.0016, Phentermin 0.0039, Norephedrin <0.001, N,N'-Dibenzylethylendiamin 0.0055, p-Hydroxymethamphetamin 0.014, p-Hydroxyamphetamin <0.001, p-Hydroxyephedrin 0.0031, p-Hydroxynorephedrin <0.001, Meskalin 0.00001.

5. Monoklonaler Antikörper gegen Methamphetamin, nach einem der Ansprüche 1 bis 4, dadurch gekennzeichnet, daß der monoklonale Antikörper mit wenigstens einem Enzym markiert ist.

6. Monoklonaler Antikörper gegen Methamphetamin, nach Anspruch 5, dadurch gekennzeichnet, daß das Enzym ausgewählt ist unter Oxidoreduktase, Peroxidase, Katalase, Glukoseoxidase, Laktatoxidase, Alkoholoxidase, Monoaminoxidase, β-Galaktosidase und Alkalinphosphatase.

7. Verfahren zum Testen von Methamphetamin, gekennzeichnet durch die Stufe, daß einem mit Methamphetamin gebundenen makromolekularen Protein, immobilisiert auf einem Festphasenträger, und Methamphetamin in einer zu testenden Probe erlaubt wird, mit einem Reagenz konkurrierend zu reagieren, welches einen mit einem Enzym markierten monoklonalen Antikörper nach Anspruch 5 oder Anspruch 6 umfaßt.

8. Methamphetamin-Testkit zur Verwendung bei dem Verfahren nach Anspruch 7, mit
(a) einem mit Methamphetamin gebundenen makromolekularen Protein; und
(b) einem Reagenz, welches einen mit einem Enzym markierten monoklonalen Antikörper nach Anspruch 5 oder Anspruch 6 umfaßt.

9. Verfahren zum Testen von Methamphetamin, gekennzeichnet durch die Stufen des Immobilisierens eines monoklonalen Antikörpers nach einem der Ansprüche 1 bis 4 auf einem Festphasenträger und der Zulassung einer Reaktion von mit einem Enzym markiertem Methamphetamin und Methamphetamin in einer zu testenden Probe konkurrierend mit dem monoklonalen Antikörper, der auf dem Festphasenträger immobilisiert wurde.

10. Methamphetamin-Testkit zur Verwendung bei dem Verfahren nach Anspruch 9, mit
(a) einem monoklonalen Antikörper nach einem der Ansprüche 1 bis 4 und
(b) Methamphetamin, das mit einem Enzym markiert ist.

11. Methamphetamin-Testkit mit
(a) einem Träger, auf welchem ein monoklonaler Antikörper nach einem der Ansprüche 1 bis 4 angeordnet ist, und
(b) einem Träger, auf welchem Methamphetamin angeordnet ist.

12. Verfahren zum Testen von Methamphetamin, gekennzeichnet durch die Verwendung eines Kits nach Anspruch 11 und durch die Stufen der Zulassung einer Reaktion von Methamphetamin in einer zu testenden Probe konkurrierend mit dem auf dem Träger angeordneten monoklonalen Antikörper.

## Revendications

1. Anticorps monoclonaux de méthamphétamine obtenus à partir de souches d'hybridomes déposées au Fermentation Research Institute, Agency of Industrial Science and Technology et ayant les numéros d'accession FERM BP-1494, FERM BP-1495 ou FERM BP-1496.

2. Anticorps monoclonal de méthamphétamine caractérisé par les réactivités croisées suivantes exprimées en termes de taux réactifs par rapport à la méthamphétamine :
méthamphétamine 1, amphétamine 0,0051, éphédrine 0,0018, méthyléphédrine 0,022, méthoxyphénamine 0,023, phentermine 0,042, noréphédrine <0,001, N,N'-dibenzyiéthylènediamine 0,17, p-hydroxyméthamphétamine 0,0039, p-hydroxyamphétamine < 0,001, p-hydroxyéphédrine <0,001, p-hydroxynoréphédrine <0,001, mescaline <0,00001.

3. Anticorps monoclonal de méthamphétamine caractérisé par les réactivités croisées suivantes exprimées en termes de taux réactifs par rapport à la méthamphétamine :-
méthamphétamine 1, amphétamine 0,0062, éphédrine 0,0025, méthylephédrine 0,028, méthoxyphénamine 0,0037, phentermine 0,12, noréphédrine <0,001, N,N'-dibenzyléthylènediamine 0,23, p-hydroxyméthamphétamine 0,14, p-hydroxyamphétamine 0,003, p-hydroxéphédrine <0,001,p-hydroxynoréphédrine <0,001, mescaline <0,00001.

4. Anticorps monoclonal de méthamphétamine caractérisé par les réactivités croisées suivantes exprimées en termes de taux réactif par rapport à la méthamphétamine : -
méthamphétamine 1, amphétamine 0,019, éphédrine 0,0012, méthyléphédrine 0,016, méthoxyphénamine 0,0016, phentermine 0,0039, noréphédrine <0,001, N,N'-dibényléthylènediamine 0,0055, p-hydroxyméthamphétamine 0,014, p-hydroxyamphétamine <0,001, p-hydroxyéphédrine 0,0031, p-hydroxynoréphédrine <0,001, mescaline <0,0001.

5. Anticorps monoclonal de méthamphétamine selon l'une quelconque des revendications 1 à 4 caractérisé en ce que l'anticorps monoclonal est marqué avec au moins une enzyme.

6. Anticorps monoclonal de méthamphétamine selon la revendication 5 caractérisé en ce que l'enzyme est choisie parmi oxydoréductase, peroxydase, catalase, glucose oxydase, lactate oxydase, alcool oxydase, monoamine oxydase, β-galactosidase, et phosphatase alcaline.

7. Méthode d'essai de méthamphétamine caractérisé par l'étape qui consiste à permettre à une protéine macromoléculaire liée à une méthamphétamine immobilisée sur un support en phase solide et à une méthamphétamine dans un échantillon d'être testées, pour réagir de façon compétitive avec un réactif comprenant un anticorps monoclonal marqué avec une enzyme selon les revendications 5 ou 6.

8. Trousse d'essai de méthamphétamine à utiliser dans la méthode décrite dans la revendication 7, comprenant,
(a) une protéine macromoléculaire liée à une méthamphétamine ; et
(b) un réactif comprenant un anticorps monoclonal marqué avec une enzyme selon les revendications 5 ou 6.

9. Méthode d'essai de méthamphétamine caractérisée par les étapes consistant à immobiliser un anticorps monoclonal selon l'une quelconque des revendications 1 à 4, sur un support en phase solide, et à permettre à une méthamphétamine marquée avec une enzyme et à une méthamphétamine dans un échantillon d'être testées pour réagir de façon compétitive avec l'anticorps monoclonal immobilisé sur le support en phase solide.

10. Trousse d'essai de méthamphétamine à utiliser dans la méthode selon la revendication 9, comprenant :
(a) un anticorps monoclonal selon l'une quelconque des revendications 1 à 4 ; et
(b) une méthamphétamine marquée avec une enzyme.

11. Trousse d'essai de méthamphétamine comprenant :
(a) un support portant sur lui un anticorps monoclonal selon l'une quelconque des revendications 1 à 4; et
(b) un support portant sur lui une méthamphétamine.

12. Méthode d'essai de méthamphétamine caractérisée par l'utilisation d'une trousse selon la revendication 11 et comprenant les étapes consistant à permettre à une méthamphétamine dans un échantillon d'être testée pour réagir de façon compétitive avec l'anticorps monoclonal sur le support.
